# EUROPEAN PATENT SPECIFICATION

(11) **EP 0 394 280 B1**
(45) Date of publication and mention of the grant of the patent: **05.04.1995**
(21) Application number: 88907649.3
(22) Date of filing: 18.08.1988
(51) Int. Cl.: C07H 15/04, C07H 15/203, C12P 7/62

(54) **ESTERS OF GLYCOSIDES AND A PROCESS FOR ENZYMATIC PREPARATION THEREOF**
GLYKOSIDESTER UND VERFAHREN ZUR ENZYMATISCHEN HERSTELLUNG
Nouveaux esters glycosidiques et procédé enzymatique de préparation desdits composés

(30) Priority: 21.08.1987 DK 4388/87
(43) Date of publication of application: 31.10.1990
(73) Proprietor: NOVO NORDISK A/S, 2880 Bagsvaerd (DK)
(72) Inventor: KIRK, Ole, DK-2200 Copenhagen N (DK); BJÖRKLING, Frederik, DK-2860 Soborg (DK); GODTFREDSEN, Sven, Erik, DK-3500 Vaerlose (DK)
(74) Representative: Noergaard, Torsten
(86) International application number: DK8800135
(87) International publication number: WO8901480

(56) References cited:
- EP-A- 0 144 894
- EP-A- 0 155 931
- WO-A-86/05186
- DE-A- 2 360 367
- US-A- 2 759 922
- US-A- 3 655 645
- US-A- 4 408 041
- US-A- 4 614 718
- Journal of the American Chemical Society, Vol. 108, 1986, pages 6421-6422,HENRI M. SWEERS et al. "Enzyme-Catalyzed Regioselective Deacylation ofProtected Sugars in Carbohydrate Synthesis".

## Description

This invention relates to novel esters of glycosides and to a process for enzymatic syntheses of such compounds.

### Background of this invention

Surface active compounds constitute an exceedingly important class of industrial organic chemicals finding a wide variety of uses, e.g. as detergent for washing purposes, as emulsifiers in food and feed products, or even as functional ingredients in many personal care products as shampoos, moisturing creams etc.

Basically, surfactants are, on the molecular level, characterised by and owe their properties to presence of hydrophobic and hydrophilic regions within the individual surfactant molecules. This particular constellation can be established in numerous fashions , e.g. by combining sulphonic acid residue, a quarternised ammonium entity or a glycerol moiety with an alkyl chain as is the case in the linear alkyl surfactants, the quarternised alkyl amines, and the monoglycosides, respectively. In the actual design of such surfactant molecules major consideration is given to the detailed molecular architecture of the compounds, important issues being the precise balance between the hydrophilic and hydrophobic domains of the surfactant molecules, and the actual special arrangements of these parts of the molecules. Besides, consideration is obviously given to the possibilities of actually producing the surfactants in high yielding processes and on the basis of raw materials available at reasonable costs. The environmental issues related to the eventual loading of the surfactant into the environment are finally a matter of major concern.

Due to these considerations there have over the years been a keen interest in preparing surfactant molecules on the basis of sugars and fatty acids, e.g. as sugar esters. Such conjugates were expected to exhibit surface active properties due to the presence of the hydrophilic sugar regions and the hydrophobic fatty acid residues. The balance, and thus the precise properties of the conjugates, might be varied through changes of the nature of the sugar and the fatty acid residues; the materials would be producable from exceedingly cheap raw materials; and the surfactants, being composed of and degradable into natural constitutents, would not be harmful to the environment.

As a specific example of surfactants, reference is made to Phillip.J.Coconut stud. 5 (1980), 51 et sec., wherein coconut fatty acid esters of methyl glucopyranoside is described. However, there is no mentioning in this paper about these surfactants being used as additives to detergents. As examples of specific commercially used surfactants which are additives to detergents, Berol 065 and Berol 160 (fatty alcohol ethoxylates) manufactured by Berol AB, Sweden, can be mentioned.

Synthesis and production of pure sugar esters by conventional means have, in spite of many attempts, turned out to be quite difficult. This is due, among other things, to the presence of several chemically similar groups in the sugar molecules which, accordingly, are esterified at several positions by exposure to esterification reagents. Sugar esters prepared by chemical means are, therefore, usually mixtures of compounds differing in respect to the degree of esterification and in the positions of the acyl groups on the carbohydrate moiety of the products. Since, in addition, the chemical procedures for chemical esterification turn out to be quite cost intensive, the sugar esters so far made available on an industrial scale find a rather limited use only.

In view of the difficulties encountered in production of sugar esters by chemical means and the attractiveness of these compounds as industrial surfactants, much attention has during the recent years been devoted to the possibility of utilising enzymes for synthesis of the sugar esters. One major rational behind this interest is that enzymes are known to exhibit a high degree of regio- and enantioselectivity which might be exploited for selective esterification of one or more hydroxy groups in sugar molecules. Cheap starting materials might be utilised in enzymatic processes which might, therefore, lead to low priced sugar esters of a high quality. The enzymes envisioned as catalysts in processes of this kind are primarily lipases which catalyse hydrolysis of ester bonds and which do, therefore, in principle, also catalyse the reverse reaction, i.e. ester synthesis.

The attempts to develop efficient enzymatic syntheses of sugar esters have, however, so far, been unsuccessful. One major reason for this failure is the major polarity difference between the two substrates of the esterification reaction, the sugar and the fatty acid or a derivative thereof, and the necessity of avoiding water in the reaction medium in order to drive the enzymatic reaction in the directions of synthesis. Few good solvents for both sugars and fatty acids or their derivatives are thus available and these solvents will often inactivate enzymes.

These difficulties inherent in enzymatic synthesis of sugar esters are reflected in the cases reported as for example in US patent specification No. 4,614,718 and in J.Am.Chem.Soc. 108 (1986), 5638 - 5640 and 6421 - 6422, and 109 (1987), 3977 - 3981. These publications teach application of lipases for esterification of sorbitol and sorbital with fatty acids, and transesterification from activated esters of free fatty acids onto long chain glucosides with lipases, respectively. The poor yields, the low selectivity of the reactions and the toxicity of solvents applied in the processes revealed exclude, however, any technical utility, of the processes described.

German Offenlegungsschrift No. 2,360,367 relates to esters of polyglycosides having a degree of glycosidation of from 1.1 to 4. No enzymatic reactions are described in this patent. In addition, there is no description in this Offenlegungsschrift of the preparation of pure compounds. The esters of glycosides prepared in Examples 10 - 13 in this Offenlegungsschrift have a degree of glycosidation of 1.3, 1.3, 1.8 and 1.6, respectively, vide the difinition at the bottom of page 3 and top of page 4 in the German Offenlegungsschrift.

U.S. patent specification No. 2,759,922 relates to a process for preparing diesters, triesters and tetraesters of glycosides. This patent does not describe the preparation of pure monoesters of glycosides. No enzymatic reactions are described in this U.S patent. As appears from the data given in Examples 16 and 17 in this U.S patent, none of the products were pure monoesters of glycosides.

U.S. patent specification No. 4,614,718 does not relate to esters of glycosides but to sugar or sugar alcohol esters of higher fatty acids. According to experiments performed by the inventors of the present invention, the process patented in this U.S patent does not work.

PCT patent application having international publication No. WO 86/05186 does not relate to esters of glycosides but to a process wherein the starting material has only one free hydroxy group.

One object of this invention is to provide novel compounds.

A further object of this invention is to provide surfactants having superior effects.

A still further object of this invention is to provide surfactants which have superior effects when used in detergents.

A further object of this invention is to provide cleaning agent compositions with better cleaning effects.

A still further object of this invention is to provide a process for preparing esters of glucosides.

A further object of this invention is to provide monoesters of glucosides.

A still further object of this invention is to provide monoesters of glucosides of monosaccharides.

### Detailed practise of this invention

It has now, surprisingly, been found that compounds of the general formula I

R-COO-X-OR¹ (I)

wherein R¹ represents alkyl with 2 - 6 carbon atoms or R¹ represents one of the groups 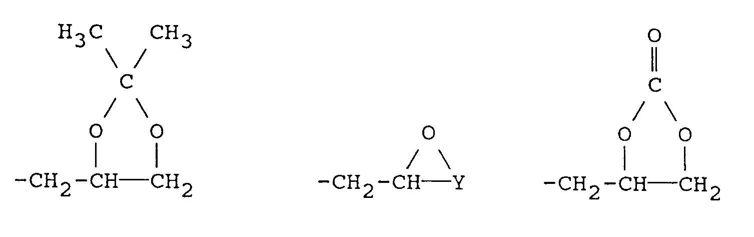
wherein Y represents methylene or ethylene, X represents a monosaccharide moiety carrying at the anomeric carbon atom the group -OR¹ and carrying a RCOO- group at a primary hydroxy group, and R represents alkyl with 4 - 24 carbon atoms, can be obtained by condensing an acid or an ester of the general formula II

R-COOR² (II)

wherein R is as stated above, and R² represents hydrogen or lower alkyl, with a glycoside of the general formula III

HO-X-OR¹ (III)

wherein X and R¹ each is as defined above, in the presence of a catalyst, said catalyst being a hydrolase. The parent carbohydrates as well as the compounds of formula I may be the α- or β-form.

The above alkyl groups may be linear or branched. The term "lower" indicates, when used in connection with alkyl groups and similar groups, that the alkyl group in question contains not more than 8 carbon atoms, preferably not more than 4 carbon atoms.

Preferably R¹ is an unsubstituted alkyl group. Preferably, the group R¹ contains 2, 3 or 4 carbon atoms. Examples of specific, preferred groups R¹ are ethyl, propyl, isopropyl and butyl, most preferred ethyl and isopropyl.

The process described below is expected to be the only process known by which it is possible to prepare acceptable yields of monoesters. Hence, monoesters of formula I have not previously been available.

Preferably X is a carbohydrate moiety containing one hexose or pentose unit, the parent carbohydrate being a monosaccharide. Preferred carbohydrates corresponding to the moiety X are glucose, fructose, ribose, arabinose, mannose, galactose and xylose, the most preferred carbohydrates being glucose and galactose.

Preferred fatty acids corresponding to the moiety R-COO- in formula I are saturated and unsaturated fatty acids, preferably containing 6 - 22 carbon atoms.

Examples of such fatty acids are hexanoic acid, heptanoic acid, octanoic acid, nonanoic acid, decanoic acid, dodecanoic acid, tetradecanoic acid, hexadecanoic acid, octadecanoic acid, eicosanoic acid, docosanoic acid, cis-9-octadecanoic acid, cis,cis-9,12-octadecanoic acid and cis,cis,cis-9,12,15-octadecatrienoic acid.

Examples of specific, preferred compounds of formula I are as follows:
Ethyl 6-O-hexanoylglucoside, ethyl 6-O-heptanoylglucoside, ethyl 6-O-octanoylglucoside, ethyl 6-O-nonanoylglucoside, ethyl 6-O-decanoylglucoside, ethyl 6-O-dodecanoylglucoside, ethyl 6-O-tetradecanoylglucoside, ethyl 6-O-hexadecanoylglucoside, ethyl 6-O-octadecanoylglucoside, ethyl 6-O-eicosanoylglucoside, ethyl 6-O-docosanoylglucoside, ethyl 6-O-cis-9-octadecanoylglucoside, ethyl 6-O-cis,cis-9,12-octadecanoylglucoside, ethyl 6-O-cis,cis,cis-9,12,15-octadecatrienoylglucoside, isopropyl 6-O-hexanoylglucoside, isopropyl 6-O-heptanoylglucoside, isopropyl 6-O-octanoylglucoside, isopropyl 6-O-nonanoylglucoside, isopropyl 6-O-decanoylglucoside, isopropyl 6-O-dodecanoylglucoside, isopropyl 6-O-tetradecanoylglucoside, isopropyl 6-O-hexadecanoylglucoside, isopropyl 6-O-octadecanoylglucoside, isopropyl 6-O-eicosanoylglucoside, isopropyl 6-O-docosanoylglucoside, isopropyl 6-O-cis-9-octadecanoylglucoside, isopropyl 6-O-cis,cis-9,12-octadecanoylglucoside, isopropyl 6-O-cis,cis,cis-9,12,15-octadecatrienoylglucoside, propyl 6-O-hexanoylglucoside, propyl 6-O-heptanoylglucoside, propyl 6-O-octanoylglucoside, propyl 6-O-nonanoylglucoside, propyl 6-O-decanoylglucoside, propyl 6-O-dodecanoylglucoside, propyl 6-O-tetradecanoylglucoside, propyl 6-O-hexadecanoylglucoside, propyl 6-O-octadecanoylglucoside, propyl 6-O-eicosanoylglucoside, propyl 6-O-docosanoylglucoside, propyl 6-O-cis-9-octadecanoylglucoside, propyl 6-O-cis,cis-9,12-octadecanoylglucoside and propyl 6-O-cis,cis,cis-9,12,15-octadecatrienoylglucoside, butyl 6-O-hexanoylglucoside, butyl 6-O-heptanoylglucoside, butyl 6-O-octanoylglucoside, butyl 6-O-nonanoylglucoside, butyl 6-O-decanoylglucoside, butyl 6-O-dodecanoylglucoside, butyl 6-O-tetradecanoylglucoside, butyl 6-O-hexadecanoylglucoside, butyl 6-O-octadecanoylglucoside, butyl 6-O-eicosanoylglucoside, butyl 6-O-docosanoylglucoside, butyl 6-O-cis-9-octadecanoylglucoside, butyl 6-O-cis,cis-9,12-octadecanoylglucoside and butyl 6-O-cis,cis,cis-9,12,15-octadecatrienoylglucoside.

The compounds of formula I have surprisingly good effects as surfactants which, for example, can be illustrated by their cleaning effects, especially towards fatty and protein soiling.

The cleaning agents containing a compound of formula I may be in any convenient form, such as powders or liquids.

Typical examples of cleaning agents are laundry detergents, dishwash detergent and hard-surface cleaner. More specific examples are liquid heavy-duty detergents (with or without builders) and powder heavy-duty detergents (with or without phosphate builders).

The surfactant of formula I in the cleaning agents may be mainly of the non-ionic hype (e.g. above 80%), or may be a combination of non-ionic (e.g. 20 - 80%) and another type of surfactant (e.g. 20 - 80% of anionic, cationic and/or zwitterionic). Examples of anionics are linear alkyl benzene sulphonates (LAS), fatty alcohol sulphates, fatty alcohol ether sulphates (AES), alpha-olefin sulphonates (AOS) and soaps.

Liquid and powder detergents may be formulated in analogy with "Frame formulations for liquid/powder heavy-duty detergents" (J. Falbe: Surfactants in Consumer Products. Theory, Technology and Application, Springer-Verlag 1987) by replacing all or part (e.g. 50%) of the non-ionic surfactant with a compound of formula I. Thus, liquid heavy-duty detergents may in addition to the compound of formula I comprise anionic surfactants, non-ionic surfactants, suds controlling agents, foaming boosters, enzymes, builders, formulation aids, optical brighteners, stabilizers, fabric softeners, fragrances, dyestuffs and water. Similarly, powder heavy-duty detergents may comprise anionic surfactants, non-ionic surfactants, suds controlling agents, foaming boosters, chelating agents, ion exchangers, alkalis, cobuilders, bleaching agents, bleach activators, bleach stabilizers, fabric softeners, antiredeposition agents, enzymes, optical brighteners, anticorrosion agents, fragrances, dyestuffs and blueing agents, formulation aids, fillers and water.

The superior effects of compounds of formula I are illustrated by example 37 below.

In addition, it has surprisingly been found that it is possible to prepare compounds of formula I by enzymatic syntheses of sugar esters in very high yields by using as substrates for the enzymatic esterification a carbohydrate carrying an alkyl group with 2 - 6 carbon atoms at the hydroxy group at the terminal anomeric carbon atom and a free fatty acid or an ester thereof as the other substrate for the reaction. The products of the reaction are sugar esters carrying an alkyl group at the hydroxy group at the anomeric carbon atom. It is highly surprising, and it could not have been predicted, that a minor change of the sugar molecules at their anomeric carbons improves their behaviour as substrates dramatically and gives high yields and even regiospecific enzymatic esterifications.

Using this process it is possible to prepare a preparation containing more than 80%, preferably more than 90%, even more preferred more than 95%, of a compound of formula I. Such a preparation has superior, surprising properties as surfactant.

The enzymes which can be applied by the process of this invention are enzymes capable of forming ester linkages. This group of enzymes comprises hydrolases, such as esterases, and lipases. The enzymes applied by the process of this invention may be used in a soluble state or the enzymes may be immobilised, if desired. Also, the enzymes may be modified by chemical or genetic methods in order to optimise their reactivity in regard to a specific reaction of interest.

Examples of specific enzymes which may be used by the process of this invention are porcine pancreatic lipase and microbial lipases obtained, e.g. from strains of Aspergillus, Rhizopus, Pseudomonas, Enterobacterium, Chromobacterium, Geotricium, Penicillium, Mucor, Candida, and Humicula. Examples of preferred strains are Mucor Miehei, Candida antarctica, Pseudomonas cepacia and Humicola lanuginosa.
Candida antarctica was deposited at Deutsche Sammlung von Mikroorganismen (DSM) according to the Budapest Treaty under the deposit Nos. DSM 3855, DSM 3908 and DSM 3909 at the 29 September 1986, 8 December 1986 and 8 December 1986, respectively.

Pseudomonas cephacia was deposited at DSM under No. 3959 on 30 January 1987 and Humicola lanuginosa was deposited at DSM under the Nos. 3819 and 4109 on 13 August 1986 and 4 May 1987, respectively.

Further lipases are obtainable from Humicola brevispora, brevis var. thermoidea and insolens which were deposited at DSM under the Nos. 4110, 4111 and 1800, respectively, on 4 May 1987, 4 May 1987 and 1 October 1981, respectively.

Additional lipases are obtainable from the following strains, which are freely available to the public from Centralbureau voor Schimmelculturen (CBS), American Type Culture Collection (ATCC), Agricultural Research Culture Collection (NRRL) and Institute of Fermentation, Osaka (IFO) under the indicated deposit numbers: Candida antarctica: CBS 5955, ATCC 34888, NRRL Y-8295, CBS 6678, ATCC 28323, CBS 6821 and NRRL Y-7954; Candida tsukubaensis: CBS 6389, ATCC 24555 and NRRL Y-7792; Candida auriculariae: CBS 6379, ATCC 24121 and IFO 1580; Candida humicola: CBS 571, ATCC 14438, IFO 0760, CBS 2041, ATCC 9949, NRRL Y-1266, IFO 0753 and IFO 1527 and Candida foliorum: CBS 5234 and ATCC 18820.

The process of this invention may be carried out simply by mixing the glycoside of formula III with an acid or an ester thereof of formula II in the presence of the enzyme and, optionally, the reaction may be carried out in a solvent in which the enzyme exhibits the desired activity. Preferably, no solvent is added. If an organic solvent is used, it should have no deleterious effect on the enzyme. Examples of such solvents are ketones, hydrocarbons and ethers. Preferred solvents are pentan, hexan, heptan and 2-butanone.

Preferably, the reaction medium is non-aqueous or contains only the approximate amount of water which is needed to ensure a good reactivity and life-time of the applied enzyme.

Conveniently, the reaction temperature is in the range of about 20 - 100°C, preferably about 30 - 80°C. Preferably, the reaction is performed at a low pressure, preferably below about 0.05 bar.

This invention is illustrated by the following examples which, however, are not to be considered as limiting in any way the scope of protection.

### General procedures

¹H and ¹³C NMR-spectra were recorded on a Bruker WM 400 and a Bruker AM 500 spectrometer with TMS as internal reference. Optical rotation was mesured on a Perkin-Elmer 241 polariometer, using 1 dm cuvette. Melting points are uncorrected. HPLC-analysis was performed on a Shimadzu LC-4A instrument (refractive index detector) using a Merck LiChrosorb NH₂-column and 96% ethanol as eluent. Critical micelle concentrations were measured on a Krüss Tensiometer K10. Molecular distillation was performed on a KDL 1 unit from Leybold-Heraeus. Isopropyl α-D-glucopyranoside n-propyl β-D-glucopyranoside and phenyl α-D-glucopyranoside were obtained as a gift from The Technical University of Denmark, Department of Organic Chemistry. Preparative liquid chromatography was performed on SiO₂ with a gradient of hexane-ethylacetate and methanol as eluent.

### Example 1

### Preparation of ethyl 6-O-dodecanoyl-D-glucopyranoside

To a mixture of crude ethyl D-glucopyranoside (578 g, 2.78 mol, prepared according to example 10) and dodecanoic acid (751 g, 3.75 mol) in a stirred batch reactor at 70°C was added immobilized lipase derived from Candida antarctica (29 g, prepared as described in Danish patent application No. 3250/88). Stirring was continued under reduced pressure (0.05 bar) and the progress of the ester synthesis was monitored by HPLC. After 23 hours the enzyme was removed by filtration (at 70°C). Excess fatty acid was removed by repeated molecular distillation (105°C, 4 · 10⁻² mbar) yielding 96% (1050 g) of crude product along with 2% ethyl D-glucoside and 2% of a mixture of diesters (HPLC analysis). The crude product was purified by chromatography. Identification by ¹H NMR-analysis showed a 1:1 mixture of α and β anomers (table 4).

### Example 2

### Preparation of ethyl 6-O-decanoyl-D-glucopyranoside

The title compound was obtained as a crude product (1030 g, 93% monoester, 5% ethyl D-glucopyranoside, 2% diesters) according to example 1 using ethyl D-glucopyranoside (625 g, 3.0 mol), decanoic acid (646 g, 3.75 mol) and immobilized lipase (31.5 g). The reaction was complete in 48 hours. NMR-spectra of the chromatographically purified product are given in table 4.

### Example 3

### Preparation of ethyl 6-O-tetradecanoyl-D-glucopyranoside

The title compound was obtained as a crude product (1160 g, 93% monoester, 4% ethyl D-glucopyranoside, 3% diesters) according to example 1 using ethyl D-glucopyranoside (609 g, 2.9 mol), tetradecanoic acid (834 g, 3.7 mol) and immobilized lipase (30.5 g). The reaction was complete in 46 hours. NMR-spectra of the chromatographically purified product are in accordance with the ¹H and ¹³C NMR-spectra given for the pure α and β anomers in table 4 a/4 b.

### Example 4

### Preparation of ethyl 6-O-hexadecanoyl-D-glucopyranoside

The title compound was obtained as a crude product (1220 g, 91% monoester, 7% ethyl D-glucopyranoside, 2% diesters) according to example 1 using ethyl D-glucopyranoside (603 g, 2.9 mol), hexadecanoic acid (1001 g, 3.91 mol) and immobilized lipase (30.5 g). The reaction was complete in 48 hours. NMR-spectra of the chromatographically purified product are in accordance with the ¹H and ¹³C NMR-spectra given for the pure α and β anomers in table 4 a/4 b.

### Example 5

### Preparation of ethyl 6-O-(cis-9-octadecenoyl)-D-glucopyranoside

The title compound was obtained as a crude product (1305 g, 90% monoester, 5% ethyl D-glucopyranoside, 5% diesters) according to example 1 using ethyl D-glucopyranoside (606 g, 2.9 mol), cis-9-octadecenoic acid (1111 g, 3.9 mol) and immobilized lipase (30.5 g). The reaction was complete in 48 hours.

### Example 6

### Preparation of ethyl 6-O-octadecanoyl-D-glucopyranoside

The title compound was obtained as a crude product (1310 g, 90% monoester, 5% ethyl D-glucopyranoside, 5% diesters) according to example 1, using a reaction temperature of 80°C, ethyl D-glucopyranoside (603 g, 2.9 mol), octadecanoic acid (1112 g, 3.9 mol) and immobilized lipase (30 g). The reaction was complete in 48 hours. NMR-spectra of the chromatographic-ally purified product are in accordance with the ¹H and ¹³C NMR-spectra given for the pure α and β anomers in table 4 a/4 b.

### Example 7

### Preparation of coconut oil fatty acid 6-O esterified ethyl D-glucopyranoside

Ethyl D-glucopyranoside (600 g, 2.9 mol) was esterified with a mixture of coconut oil fatty acids (containing 1% decanoic acid, 51% dodecanoic acid, 24% tetradecanoic acid, 13% hexadecanoic acid, 4% octadecanoic acid, 5% cis-9-octadecenoic acid and 2% cis,cis-9,12-octadecadienoic acid in a total amount of 3.0 mol) by the procedure described in example 1 using 30 g of immobilized lipase as catalyst. After 72 hours the reaction was complete yielding 1200 g of product (91% monoesters, 6% diesters and 3% ethyl D-glucopyranoside.

### Example 8

### Preparation of ethyl 6-O-otanoyl-D-glucopyranoside

To a suspension of ethyl D-glucopyranoside (500 g, 2.4 mol prepared according to example 10) and octanoic acid (520 g, 3.6 mol) in hexane (1000 ml) in a stirred batch reactor at 70°C (reflux) was added immobilized lipase (5 g Lipozyme™, commercial available NOVO lipase prepared from a Mucor Miehei). Stirring was continued and the produced water was removed by azeotropic distillation. The progress of the reaction was followed by HPLC. As the product was being formed, the suspension gradually became a homogeneous solution (after 12 hours). After 52 hours the enzyme was removed by filtration and the solvent removed in vacuo. Excess fatty acid was removed by repeated molecular distillation giving a crude product (790 g, 91% monoester, 8% diester and 1% ethyl D-glucopyranoside). H¹ and ¹³C NMR-spectra of the purified product were in accordance with the spectra for the pure α-and β-anomers given in table 4 a/4 b.

### Example 9

### Preparation of isopropyl 6-O-dodecanoyl-D-glucopyranoside

The title compound was prepared according to example 1 using isopropyl D-glucopyranoside (446 g, 2.01 mol prepared according to example 11). After 24 hours the reaction was complete and a crude product isolated (561.1 g, 73.4% monoester and 9.2% diester).

### Example 10

### Preparation of ethyl D-glucopyranoside

Glucose (500 g, 2.78 mol) and a strong acid cation exchange resin (100 g Amberlyst 15, BDH Chemicals) was suspended in ethanol (2000 ml, 34.3 mol). The mixture was stirred at 80°C for 16 hours. The progress of the reaction was followed by HPLC. The ion exchange resin was removed by filtration and the solution was treated with activated carbon (10 g). After filtration the ethanol was removed in vacuo giving ethyl D-glucopyranoside (a 1:1 mixture of α and β anomers) as a syrup (578 g, quantitative yield).

### Example 11

### Preparation of isopropyl D-glucopyranoside

The title compound was prepared in quantitative yield by the procedure described in example 10 using isopropanol (2000 ml, 26 mol) and glucose (500 g, 2.78 mol).

### Example 12 - 17

### Preparation of 6-O-esters of pure β-D-glucopyranoside

### General procedure:

Ethyl β-D-glucopyranoside (3.0 g, 14 mmol prepared according to example 24) was dissolved/suspended in melted fatty acid (typical 28 mmol) at 70°C. Immobilized lipase (typical 0.5 g Lipozyme™, see example 8) was added and the mixture was stirred at reduced pressure (0.05 bar). The progress of the reaction was followed by HPLC. The reaction of this mixture was diluted with acetone and the enzyme was removed by filtration. The solvent was removed in vacuo and the product was recovered by chromatography on SiO₂. For ¹H and ¹³C NMR (see table 4 a) it was further characterised by melting points (m.p.), optical rotation ([α]_{D}) and critical micelle concentration (CMC) measurement (given in table 3).

The experimental details from the different examples are shown in table 1.

**TABLE 1**

| Example | Fatty acid | | | Lipozyme | Reaction time | Yield |
|---|---|---|---|---|---|---|
| | | g | mmol | g | hours | % |
| 12 | Octanoic | 8.3 | 58 | 1.0 | 240 | 75 |
| 13 | Decanoic | 5.0 | 28 | 0.5 | 64 | 81 |
| 14 | Dodecanoic | 5.7 | 28 | 0.5 | 24 | 87 |
| 15 | Tetradecanoic | 6.6 | 28 | 0.5 | 22 | 83 |
| 16 | Hexadecanoic | 7.3 | 28 | 0.5 | 40 | 77 |
| 17 | Octadecanoic | 8.2 | 28 | 0.5 | 76 | 76 |

Experimental details form the preparation of 6-O-esters of pure β-D-glucopyranoside.

### Example 18 - 23

### Preparation of 6-O-esters of pure α-D-glucopyranoside

### General procedure:

Ethyl α-D-glucopyranoside (2.0 g, 9 mmol prepared according to example 25) was dissolved/suspended in melted fatty acid (typical 18 mmol). Immobilized lipase (typical 0.33 g) was added as in example 12-17. ¹H and ¹³C NMR (see table 4 b). Melting points (m.p.), optical rotation ([α]_{D}) and critical micelle concentration (CMC) measurement (these data are presented in table 3).

The experimental details from the different examples are shown in table 2.

**TABLE 2**

| Example | Fatty acid | | | Lipozyme | Reaction time | Yield |
|---|---|---|---|---|---|---|
| | | g | mmol | g | hours | % |
| 18 | Octanoic | 9.3 | 54 | 0.67 | 312 | 50 |
| 19 | Decanoic | 5.0 | 27 | 0.33 | 192 | 71 |
| 20 | Dodecanoic | 3.8 | 18 | 0.33 | 76 | 71 |
| 21 | Tetradecanoic | 4.4 | 18 | 0.33 | 36 | 76 |
| 22 | Hexadecanoic | 4.9 | 18 | 0.33 | 72 | 74 |
| 23 | Octadecanoic | 5.5 | 18 | 0.33 | 52 | 83 |

Experimental details form the preparation of 6-O-esters of pure α-D-glucopyranoside.

**TABLE 3**

| Physical data of the products from example 12-23: | | | | | |
|---|---|---|---|---|---|
| Example | Anomer α or β | Fatty acid | m.p. °C | [α]²_{D}⁵ | CMC* |
| 12 | β | Octanoic | 79-80 | -52.4 (c=1.0) | >500 |
| 18 | α | Octanoic | syrup | 70.9 (c=1.1) | >500 |
| 13 | β | Decanoic | 82-83 | -49.7 (c=1.0) | 110 |
| 19 | α | Decanoic | syrup | 63.0 (c=1.3) | 69 |
| 14 | β | Dodecanoic | 77-78 | -46.7 (c=1.1) | 6.9 |
| 20 | α | Dodecanoic | syrup | 60.7 (c=1.4) | 4.7 |
| 15 | β | Tetradecanoic | 82-83 | -43.5 (c=1.2) | 24 |
| 21 | α | Tetradecanoic | 42-45 | 54.5 (c=1.3) | 2.9 |
| 16 | β | Hexadecanoic | 90 | -42.4 (c=1.0) | 4.0 |
| 22 | α | Hexadecanoic | 46-51 | 49.9 (c=1.1) | 3.9 |
| 17 | β | Octadecanoic | 93-94 | -39.7 (c=1.0) | 2.6 |
| 23 | α | Octadecanoic | 54-56 | 48.4 (c=1.1) | **) |

| | | | | | |
|---|---|---|---|---|---|
| *) 10⁻⁵ mol/l, all measured in CHCl₃. | | | | | |
| **) The product was too insoluble. | | | | | |

### Example 24

### Preparation of ethyl β-D-glucopyranoside

To a suspension of silver carbonate (30 g, 0.11 mol) was added 2,3,4,6-tetra-O-acetyl-α-D-glucopyranosyl bromide (41.1 g, 0.1 mol) in small portions over a period of 40 min. The mixture was stirred overnight, then diluted with dichloromethane (40 ml) and filtrated through celite and activated carbon. Ethyl 2,3,4,5-tetra-O-acetyl-β-D-glucopyranoside (23.4 g, 62%) crystallized upon concentration. The ethyl 2,3,4,5-tetra-O-acetyl-glucopyranoside was deacetylated with 1 M sodium methoxide (2 ml) in Methanol (80 ml) for 20 hours at room temperature. The mixture was neutralized with Amberlite IR-120 (H⁺-form) and concentrated in vacuo to give the product in quantitative yield as a hygroscopic solid.

### Example 25

### Preparation of ethyl α-D-glucopyranoside

Ethyl D-glucopyranoside (30 g, prepared as described in example 10) with an anomeric ratio α:β = 1:1 was dissolved in 0.05 M acetate buffer (400 ml, pH 4.5) at 30°C. β-glucosidase (50 mg from almonds, Sigma) was added and the mixture was stirred for a week. The solution was evaporated in vacuo and purified by chromatography to give the product as a crystalline solid (8.2 g, 55%).

### Example 26

### Preparation of isopropyl 6-O-octanoyl-α-D-glucopyranoside

To a stirred solution of isopropyl-α-D-glucopyranoside (1.1 g, 5 mmol) and octanoic acid (0.9 g, 6.25 mmol) in 100 ml 2-butanone was added immobilized lipase (0.5 g Lipozyme™, see example 8). Stirring was continued at 60°C for 48 hours. The enzyme was removed by filtration and the solvent was removed in vacuo followed by chromatography yielding 1.2 g (70%) of the product ¹H NMR (400 MHz, CDCl₃) δ: 0.88 (t, J = 6.7 Hz, 3H); 1.19 (d, J = 6.1 Hz, 3H); 1.24 (d, J = 6.2 Hz, 3H); 1.28 (m, 8H); 1.62 (m, 2H); 2.34 (m, 2H); 3.35 (t, J = 9.4 Hz, 1H); 3.50 (dd, J = 4.0 and 9.5 Hz, 1H); 3.73 (t, J = 9.3 Hz, 1H); 3.85 (m, 1H); 3.92 (m, 1H) 4.35 (m, 2H); 4.96 (d, J = 3.9 Hz, 1H).

### Example 27

### Preparation of n-butyl 6-O-octanoyl-β-D-glucopyranoside

The title compound was prepared according to the method described in example 26 using (n-butyl)-β-D-glucopyranoside (1.0 g, 4.2 mmol) in 2-butanone (50 ml) in a 25% yield after 24 hours: ¹H NMR (400 MHZ, CDCl₃) δ: 0.90 (m, 6H); 1.29 (M, 8H); 1.37 (m, 2H); 1.61 (m, 4H); 2.35 (m, 2H); 3.35 (m, 2H); 3.50 (m, 3H); 3.85 (m, 1H); 4.27 (d, J = 8 Hz, 1H); 4.28 (m, 1H); 4.38 (m, 1H).

### Example 28

### Preparation of ethyl 6-O-octanoyl-α-D-glucopyranoside

After a reaction time of 48 hours the title compound was prepared in a 70% yield according to example 26 using an immobilized lipase from a Pseudomonas cepacia (0.5 g, prepared as described in Danish Patent application No. 3993/87) and ethyl-α-D-glucopyranoside (1.04 g, 5 mmol, prepared as described in example 25). For ¹H NMR see table 4 b.

### Example 29

### Preparation of ethyl 6-O-dodecanoyl-D-glucopyranoside

The title compound was prepared according to example 1 using 1/10 of the molar amounts and an immobilized lipase prepared from a Humicola lanuginosa (3.0 g prepared as described in Danish Patent application No. 3183.000/DK) as a catalyst yielding a crude product (100 g containing 83% monoester and 15% diester and 2% ethyl D-glucopyranoside) after a reaction time of 16 hours.

In tables 4 a and 4 b ¹H-NMR and ¹³C-NMR data for ethyl 6-O-acylglucopyranosides of formula I are given.

**TABLE 4 a**

| | | Ethyl 6-O-acyl-β-D-glucopyranosides | | | | | |
|---|---|---|---|---|---|---|---|
| | | C₈ | C₁₀ | C₁₂ | C₁₄ | C₁₆ | C₁₈ |
| C1 | | 102.3 | 102.4 | 102.3 | 102.4 | 102.3 | 102.4 |
| C2 | | 73.3 | 73.4 | 73.4 | 73.4 | 73.3 | 73.5 |
| C3 | | 76.0 | 76.3 | 76.2 | 75.9 | 76.0 | 75.8 |
| C4 | | 70.1 | 70.4 | 70.4 | 70.1 | 70.1 | 70.0 |
| C5 | | 73.8 | 73.9 | 73.8 | 73.9 | 73.8 | 74.0 |
| C6 | | 63.3 | 63.5 | 63.5 | 63.2 | 63.3 | 63.0 |
| CH₃CH₂O- | | 65.5 | 65.4 | 65.4 | 65.5 | 65.5 | 65.6 |
| CH3_{C}H₂O- | | 15.0 | 15.0 | 15.0 | 15.0 | 15.0 | 15.0 |
| COO | | 174.3 | 174.1 | 174.1 | 174.5 | 174.4 | 174.3 |
| H1 | | 4.28 | 4.28 | 4.28 | 4.29 | 4.28 | 4.28 |
| H2 | | 3.37 | 3.37 | 3.37 | 3.37 | 3.36 | 3.37 |
| H3 | | 3.55 | 3.54 | 3.54 | 3.54 | 3.54 | 3.57 |
| H4 | | 3.37 | 3.37 | 3.37 | 3.37 | 3.36 | 3.37 |
| H5 | | 3.47 | 3.47 | 3.48 | 3.48 | 3.46 | 3.46 |
| H6a | | 4.35 | 4.35 | 4.32 | 4.31 | 4.35 | 4.30 |
| H6b | | 4.35 | 4.35 | 4.37 | 4.38 | 4.35 | 4.47 |
| CH₃CH₂O- | a | 3.61 | 3.61 | 3.62 | 3.61 | 3.61 | 3.61 |
| | b | 3.94 | 3.94 | 3.94 | 3.94 | 3.94 | 3.96 |

**TABLE 4 b**

| | | Ethyl 6-O-acyl-α-D-glucopyranosides | | | | | |
|---|---|---|---|---|---|---|---|
| | | C₈ | C₁₀ | C₁₂ | C₁₄ | C₁₆ | C₁₈ |
| C1 | | 98.3 | 98.3 | 98.3 | 98.0 | 98.1 | 98.3 |
| C2 | | 72.1 | 72.2 | 72.2 | 72.0 | 71.9 | 72.1 |
| C3 | | 74.4 | 74.5 | 74.5 | 74.3 | 74.1 | 74.4 |
| C4 | | 70.6 | 70.7 | 70.6 | 70.0 | 70.1 | 70.6 |
| C5 | | 69.9 | 70.0 | 70.0 | 69.8 | 69.7 | 70.0 |
| C6 | | 63.7 | 63.7 | 63.6 | 63.1 | 63.2 | 63.6 |
| CH₃CH₂O- | | 63.9 | 63.9 | 63.9 | 63.8 | 63.7 | 63.9 |
| CH₃CH₂O- | | 15.0 | 15.0 | 15.0 | 14.9 | 14.9 | 15.0 |
| COO | | 174.2 | 174.2 | 174.2 | 174.4 | 174.3 | 174.2 |
| H1 | | 4.87 | 4.87 | 4.88 | 4.88 | 4.87 | 4.87 |
| H2 | | 3.51 | 3.51 | 3.51 | 3.52 | 3.51 | 3.51 |
| H3 | | 3.76 | 3.75 | 3.75 | 3.75 | 3.75 | 3.75 |
| H4 | | 3.35 | 3.35 | 3.34 | 3.34 | 3.34 | 3.34 |
| H5 | | 3.78 | 3.78 | 3.77 | 3.77 | 3.78 | 3.77 |
| H6a | | 4.32 | 4.32 | 4.28 | 4.31 | 4.31 | 4.29 |
| H6b | | 4.37 | 4.36 | 4.45 | 4.31 | 4.37 | 4.41 |
| CH₃CH₂O- | a | 3.57 | 3.57 | 3.56 | 3.56 | 3.56 | 3.5 |
| | b | 3.77 | 3.77 | 3.78 | 3.77 | 3.77 | 3.77 |

The columns in tables 4 a and 4 b give the data for compounds wherein the parent fatty acid (RCOOH) contains 8, 10, 12, 14, 16 and 18 carbon atoms corresponding to the column titles C₈, C₁₀, C₁₂, C₁₄, C₁₆ and C₁₈, respectively. C₁ - C₆ indicate carbons in the glucoside and H1 - H6b indicate hydrogen atoms in the glucoside for which ¹³C NMR and ¹H NMR, respectively, are given in these tables.

### Example 30

### Preparation of ethyl 6-O-octanoyl-D-galactoside

The title compound was prepared according to example 12 using ethyl D-galactoside (11.5 g, 56 mmol, prepared according to example 31), octanoic acid (16 g, 111 mmol) and Lipozyme (2 g, see example 8). After 24 hours the product was recovered in a yield of 80%.

### Example 31

### Preparation of ethyl D-galactoside

The title compound was prepared according to example 10 using galactose. The product (being a mixture of ethyl D-galactopyranoside and ethyl D-galactofuranoside) was isolated in quantitative yield.

### Example 32

### Preparation of 2,3-isopropylidene-glyceryl 6-O-hexadecanoyl-α-D-galactopyranoside

The title compound was prepared as described in example 26 in a yield of 70% after a reaction time of 16 hours.

### Example 33

### Preparation of phenyl 6-O-octanoyl-α-D-glucopyranoside

The title compound was prepared as described in example 27 in a yield of 25% after a reaction time of 24 hours.

### Example 34

### Preparation of propylene glycol 6-O-dodecanoyl-D-glucopyranoside

The title compound was prepared according to example 1, using propylene glycol D-glucopyranoside (40.0 g, 0.17 mol, prepared according to example 10 using propylene glycol, dodecanoic acid (45 g, 0.23 mol) and immobilized lipase (4 g). After a reaction time of 48 hours the crude product was isolated and purified by chromatography in a yield of 16%.

### Example 35

### Preparation ethyl 6-O-octanoyl-α-D-glucopyranoside by transesterification

The title compound was prepared according to example 26 using ethyl-α-D-glucopyranoside (1.04 g, 5 mmol) and methyl octanoate (1.8 g, 11 mmol) as substrates. After a reaction time of 24 hours the product was isolated in a yield of 10%.

### Example 36

### Preparation of ethyl di-hexadecanoyl-D-glucopyranoside

The title compound was prepared according to example 29 using immobilized Candida antarctica (6 g, see example 1) and four times the amount of dodecanoic acid (300 g, 1.5 mol). After a reaction time of 6 days the crude product showed a diester content of 50% (HPLC analysis).

### Example 37

Experiments were performed in a Terc-O-tometer using the following conditions:
Washing time: 20 min.
Temperature: 25°C
Water: 9°dH
Test material: EMPA 112 (7 · 7 cm)
Detergent:

| | |
|---|---|
| Sodium triphosphate | 1.75 g/l |
| Sodium metasilicate | 0.40 g/l |
| CMC | 0.05 g/l |
| EDTA | 0.01 g/l |
| Sodium sulphate | 2.00 g/l |
| Tensid | 0.60 g/l |

The tensid used was 75% of a linear alkyl benzene sulphonate (LAS, Nansa S80) and 25% of a compound of formula I. The results obtained appear from table 5 below:

**TABLE 5**

| Test compound | % residual fat weight/weight |
|---|---|
| Berol 065 | 2.44 |
| Berol 160 | 2.55 |
| Ethyl 6-O-octanoylglucoside | 2.38 |
| Ethyl 6-O-decanoylglucoside | 2.24 |
| Ethyl 6-O-dodecanoylglucoside | 2.30 |
| Ethyl 6-O-hexadecanoylglucoside | 2.31 |

## Claims

1. Process for preparing compounds of formula I
R-COO-X-OR¹ (I)
wherein R¹ represents alkyl with 2 - 6 carbon atoms or R¹ represents one of the groups 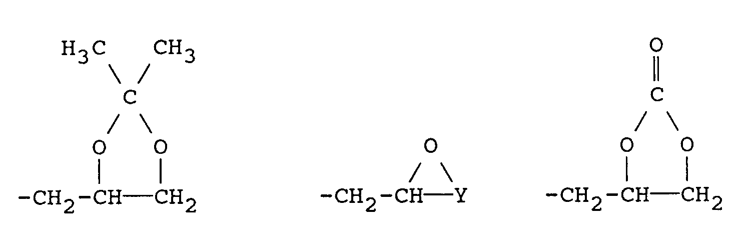 wherein Y represents methylene or ethylene, X represents a monosaccharide moiety carrying at the anomeric carbon atom the group -OR¹ and carrying a RCOO- group at a primary hydroxy group, and R represents alkyl with 4 - 24 carbon atoms, by condensing an acid or an ester of the general formula II
R-COOR² (II)
wherein R is as stated above, and R² represents hydrogen or lower alkyl, with a glycoside of the general formula III
HO-X-OR¹ (III)
wherein X and R¹ each is as defined above, in the presence of a catalyst, characterized by said catalyst being a hydrolase.

2. A process according to Claim 1 wherein the monosaccharide corresponding to the moiety X carries the group -OR¹ at the terminal anomeric carbon atom.

3. A process according to Claim 1 or 2 wherein R¹ is an unsubstituted alkyl group.

4. A process according to any one of the preceding claims wherein R¹ contains 2, 3 or 4 carbon atoms.

5. A process according to any one of the preceding claims wherein R¹ is ethyl, propyl, isopropyl or butyl, preferably ethyl or isopropyl.

6. A process according to any one of the preceding claims wherein the monosaccharide corresponding to the moiety X is glucose, fructose, ribose, mannose or galactose, preferably glucose or galactose.

7. A process according to any one of the preceding claims wherein the fatty acid corresponding to the moiety R-COO- contains 6 - 22 carbon atoms.

8. A process according to Claim 7 wherein the fatty acid is hexanoic acid, heptanoic acid, octanoic acid, nonanoic acid, decanoic acid, dodecanoic acid, tetradecanoic acid, hexadecanoic acid, octadecanoic acid, eicosanoic acid, docosanoic acid, cis-9-octadecanoic acid, cis,cis-9,12-octadecanoic acid or cis,cis,cis-9,12,15-octadecatrienoic acid.

9. A process according to Claim 8 wherein R¹ is ethyl, isopropyl, propyl or butyl, and X is a glucose moiety.

10. A process according to any one of the preceding claims characterized by the hydrolase being a lipase, an esterase or a protease.

11. Process according to any one of the preceding claims characterized by the lipase being producable by species of Mucor, Humicola, Pseudomonas or Candida.

12. Process according to any one of the preceding claims characterized by the enzyme being applied in an immobilized form.

13. Process according to any one of the preceding claims characterized in that no solvent is added.

14. Compounds of the general formula I
R-COO-X-OR¹ (I)
wherein R¹ represents alkyl with 2 - 6 carbon atoms or R¹ represents one of the groups 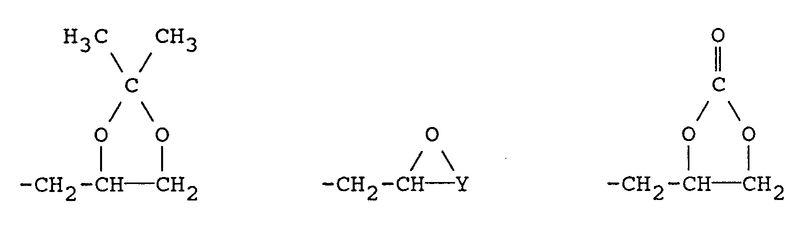 wherein Y represents methylene or ethylene, X represents a monosaccharide moiety carrying at the anomeric carbon atom the group -OR¹ and carrying a RCOO- group at a primary hydroxy group, and R represents alkyl with 4 - 24 carbon atoms.

15. Compounds according to Claim 14 wherein the monosaccharide corresponding to the moiety X carries the group -OR¹ at the terminal anomeric carbon atom.

16. Compounds according to Claim 14 or 15 wherein R¹ is an unsubstituted alkyl group.

17. Compounds according to any one of the preceding claims wherein R¹ contains 2, 3 or 4 carbon atoms.

18. Compounds according to any one of the preceding claims wherein R¹ is ethyl, propyl, isopropyl or butyl, preferably ethyl or isopropyl.

19. Compounds according to any one of the preceding claims wherein the monosaccharide corresponding to the moiety X is glucose, fructose, ribose, mannose or galactose, preferably glucose or galactose.

20. Compounds according to any one of the Claims 16 - 21 wherein the fatty acid corresponding to the moiety R-COO-contains 6 - 22 carbon atoms.

21. Compounds according to Claim 20 wherein the fatty acid is hexanoic acid, heptanoic acid, octanoic acid, nonanoic acid, decanoic acid, dodecanoic acid, tetradecanoic acid, hexadecanoic acid, octadecanoic acid, eicosanoic acid, docosanoic acid, cis-9-octadecanoic acid, cis,cis-9,12-octadecanoic acid or cis,cis,cis-9,12,15-octadecatrienoic acid.

22. Compounds according to Claim 14 being ethyl 6-O-hexanoylglucoside, ethyl 6-O-heptanoylglucoside, ethyl 6-O-octanoylglucoside, ethyl 6-O-nonanoylglucoside, ethyl 6-O-decanoylglucoside, ethyl 6-O-dodecanoylglucoside, ethyl 6-O-tetradecanoylglucoside, ethyl 6-O-hexadecanoylglucoside, ethyl 6-O-octadecanoylglucoside, ethyl 6-O-eicosanoylglucoside, ethyl 6-O-docosanoylglucoside, ethyl 6-O-cis-9-octadecanoylglucoside, ethyl 6-O-cis,cis-9,12-octadecanoylglucoside, ethyl 6-O-cis,cis,cis-9,12,15-octadecatrienoylglucoside, isopropyl 6-O-hexanoylglucoside, isopropyl 6-O-heptanoylglucoside, isopropyl 6-O-octanoylglucoside, isopropyl 6-O-nonanoylglucoside, isopropyl 6-O-decanoylglucoside, isopropyl 6-O-dodecanoylglucoside, isopropyl 6-O-tetradecanoylglucoside, isopropyl 6-O-hexadecanoylglucoside, isopropyl 6-O-octadecanoylglucoside, isopropyl 6-O-eicosanoylglucoside, isopropyl 6-O-docosanoylglucoside, isopropyl 6-O-cis-9-octadecanoylglucoside, isopropyl 6-O-cis,cis-9,12-octadecanoylglucoside, isopropyl 6-O-cis,cis,cis-9,12,15-octadecatrienoylglucoside, propyl 6-O-hexanoylglucoside, propyl 6-O-heptanoylglucoside, propyl 6-O-octanoylglucoside, propyl 6-O-nonanoylglucoside, propyl 6-O-decanoylglucoside, propyl 6-O-dodecanoylglucoside, propyl 6-O-tetradecanoylglucoside, propyl 6-O-hexadecanoylglucoside, propyl 6-O-octadecanoylglucoside, propyl 6-O-eicosanoylglucoside, propyl 6-O-docosanoylglucoside, propyl 6-O-cis-9-octadecanoylglucoside, propyl 6-O-cis,cis-9,12-octadecanoylglucoside, propyl 6-O-cis,cis,cis-9,12,15-octadecatrienoylglucoside, butyl 6-O-hexanoylglucoside, butyl 6-O-heptanoylglucoside, butyl 6-O-octanoylglucoside, butyl 6-O-nonanoylglucoside, butyl 6-O-decanoylglucoside, butyl 6-O-dodecanoylglucoside, butyl 6-O-tetradecanoylglucoside, butyl 6-O-hexadecanoylglucoside, butyl 6-O-octadecanoylglucoside, butyl 6-O-eicosanoylglucoside, butyl 6-O-docosanoylglucoside, butyl 6-O-cis-9-octadecanoylglucoside, butyl 6-O-cis,cis-9,12-octadecanoylglucoside or butyl 6-O-cis,cis,cis-9,12,15-octadecatrienoylglucoside.

23. A product containing more than 80%, preferably more than 90%, most preferred more than 95%, of a compound of formula I according to any one of the claims 14 - 22.

24. A cleaning agent containing a compound of formula I according to any one of the claims 14 - 22.

## Patentansprüche

1. Verfahren zur Herstellung von Verbindungen von Formel I
R-COO-X-OR¹ (I),
in der R¹ Alkyl mit 2 - 6 Kohlenstoffatomen darstellt oder R¹ eine der Gruppen darstellt 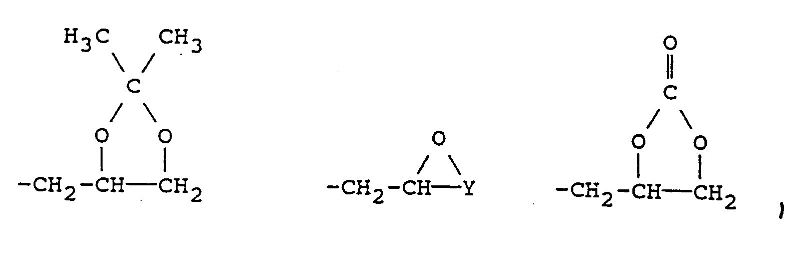 wobei Y Methylen oder Ethylen darstellt, X eine Monosaccharideinheit darstellt, die am anomeren Kohlenstoffatom die Gruppe -OR¹ trägt und eine Gruppe RCOO- an einer primären Hydroxygruppe trägt, und R Alkyl mit 4 - 24 Kohlenstoffatomen darstellt, durch Kondensieren einer Säure oder eines Esters der allgemeinen Formel II
R-COOR² (II),
in der R wie oben angegeben ist und R² Wasserstoff oder Niederalkyl darstellt, mit einem Glykosid der allgemeinen Formel III
HO-X-OR¹ (III),
in der X und R¹ jedes wie oben definiert sind, in der Gegenwart eines Katalysators, dadurch gekennzeichnet, daß besagter Katalysator eine Hydrolase ist.

2. Ein Verfahren nach Anspruch 1, wobei das Monosaccharid, das der Einheit X entspricht, die Gruppe -OR¹ am endständigen anomeren Kohlenstoffatom trägt.

3. Ein Verfahren nach Anspruch 1 oder 2, wobei R¹ eine unsubstituierte Alkylgruppe ist.

4. Ein Verfahren nach einem der vorangehenden Ansprüche, wobei R¹ 2, 3 oder 4 Kohlenstoffatome enthält.

5. Ein Verfahren nach einem der vorangehenden Ansprüche, wobei R¹ Ethyl, Propyl, Isopropyl oder Butyl, vorzugsweise Ethyl oder Isopropyl ist.

6. Ein Verfahren nach einem der vorangehenden Ansprüche, wobei das Monosaccharid, das der Einheit X entspricht, Glucose, Fructose, Ribose, Mannose oder Galactose, vorzugsweise Glucose oder Galactose ist.

7. Ein Verfahren nach einem der vorangehenden Ansprüche, wobei die Fettsäure, die der Einheit R-COO- entspricht, 6 - 22 Kohlenstoffatome enthält.

8. Ein Verfahren nach Anspruch 7, wobei die Fettsäure Hexansäure, Heptansäure, Octansäure, Nonansäure, Decansäure, Dodecansäure, Tetradecansäure, Hexadecansäure, Octadecansäure, Eicosansäure, Docosansäure, cis-9-Octadecansäure, cis,cis-9,12-Octadecansäure oder cis,cis,cis-9,12,15-Octadecatriensäure ist.

9. Ein Verfahren nach Anspruch 8, wobei R¹ Ethyl, Isopropyl, Propyl oder Butyl ist und X eine Glucoseeinheit ist.

10. Ein Verfahren nach einem der vorangehenden Ansprüche, dadurch gekennzeichnet, daß die Hydrolase eine Lipase, eine Esterase oder eine Protease ist.

11. Verfahren nach einem der vorangehenden Ansprüche, dadurch gekennzeichnet, daß die Lipase von Spezies von Mucor, Humicola, Pseudomonas oder Candida produziert werden kann.

12. Verfahren nach einem der vorangehenden Ansprüche, dadurch gekennzeichnet, daß das Enzym in einer immobilisierten Form angewendet wird.

13. Verfahren nach einem der vorangehenden Ansprüche, dadurch gekennzeichnet, daß kein Lösungsmittel zugegeben wird.

14. Verbindungen der allgemeinen Formel I
R-COO-X-OR¹ (I),
in der R¹ Alkyl mit 2 - 6 Kohlenstoffatomen darstellt oder R¹ eine der Gruppen darstellt 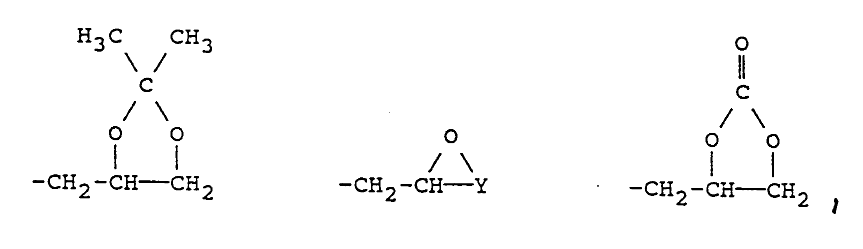 wobei Y Methylen oder Ethylen darstellt, X eine Monosaccharideinheit darstellt, die am anomeren Kohlenstoffatom die Gruppe -OR¹ trägt und eine Gruppe RCOO- an einer primären Hydroxygruppe trägt, und R Alkyl mit 4 - 24 Kohlenstoffatomen darstellt.

15. Verbindungen nach Anspruch 14, wobei das Monosaccharid, das der Einheit X entspricht, die Gruppe -OR¹ am endständigen anomeren Kohlenstoffatom trägt.

16. Verbindungen nach Anspruch 14 oder 15, wobei R¹ eine unsubstituierte Alkylgruppe ist.

17. Verbindungen nach einem der vorangehenden Ansprüche, wobei R¹ 2, 3 oder 4 Kohlenstoffatome enthält.

18. Verbindungen nach einem der vorangehenden Ansprüche, wobei R¹ Ethyl, Propyl, Isopropyl oder Butyl, vorzugsweise Ethyl oder Isopropyl ist.

19. Verbindungen nach einem der vorangehenden Ansprüche, wobei das Monosaccharid, das der Einheit X entspricht, Glucose, Fructose, Ribose, Mannose oder Galactose, vorzugsweise Glucose oder Galactose ist.

20. Verbindungen nach einem der Ansprüche 16 - 21, wobei die Fettsäure, die der Einheit R-COO- entspricht, 6 - 22 Kohlenstoffatome enthält.

21. Verbindungen nach Anspruch 20, wobei die Fettsäure Hexansäure, Heptansäure, Octansäure, Nonansäure, Decansäure, Dodecansäure, Tetradecansäure, Hexadecansäure, Octadecansäure, Eicosansäure, Docosansäure, cis-9-Octadecansäure, cis,cis-9,12-Octadecansäure oder cis,cis,cis-9,12,15-Octadecatriensäure ist.

22. Verbindungen nach Anspruch 14, die Ethyl-6-O-hexanoylglucosid, Ethyl-6-O-heptanoylglucosid, Ethyl-6-O-octanoylglucosid, Ethyl-6-O-nonanoylglucosid, Ethyl-6-O-decanoylglucosid, Ethyl-6-O-dodecanoylglucosid, Ethyl-6-O-tetradecanoylglucosid, Ethyl-6-O-hexadecanoylglucosid, Ethyl6-O-octadecanoylglucosid, Ethyl-6-O-eicosanoylglucosid, Ethyl-6-O-docosanoylglucosid, Ethyl-6-O-cis-9-octadecanoylglucosid, Ethyl-6-O-cis,cis-9,12-octadecanoylglucosid, Ethyl-6-O-cis,cis,cis-9,12,15-octadecatrienoylglucosid, Isoproyl-6-O-hexanoylglucosid, Isopropyl-6-O-heptanoylglucosid, Isopropyl-6-O-octanoylglucosid, Isopropyl-6-O-nonanoylglucosid, Isopropyl-6-O-decanoylglucosid, Isopropyl-6-O-dodicanoylglucosid, Isopropyl-6-O-tetradecanoylglucosid, Isopropyl-6-O-hexadecanoylglucosid, Isopropyl-6-O-octadecanoylglucosid, Isopropyl-6-O-eicosanoylglucosid, Isopropyl-6-O-docosanoylglucosid, Isopropyl6-O-cis-9-octadecanoylglucosid, Isopropyl-6-O-cis,cis-9,12-octadecanoylglucosid, Isopropyl-6-O-cis,cis,cis-9,12,15-octadecatrienoylglucosid, Propyl-6-O-hexanoylglucosid, Propyl-6-O-heptanoylglucosid, Propyl-6-O-octanoylglucosid, Propyl-6-O-nonanoylglucosid, Propyl-6-O-decanoylglucosid, Propyl-6-O-dodecanoylglucosid, Propyl-6-O-tetradecanoylglucosid, Propyl-6-O-hexadecanoylglucosid, Propyl-6-O-octadecanoylglucosid, Propyl-6-O-eicosanoylglucosid, Propyl-6-O-docosanoylglucosid, Propyl-6-O-cis-9-octadecanoylglucosid, Propyl-6-O-cis,cis-9,12-octadecanoylglucosid, Propyl-6-O-cis,cis,cis-9,12,15-octadecatrienoylglucosid, Butyl-6-O-hexanoylglucosid, Butyl-6-O-heptanoylglucosid, Butyl-6-O-octanoylglucosid, Butyl-6-O-nananoylglucosid, Butyl-6-O-decanoylglucosid, Butyl-6-O-dodecanoylglucosid, Butyl-6-O-tetradecanoylglucosid, Butyl-6-O-hexadecanoylglucosid, Butyl-6-O-octadecanoylglucosid, Butyl-6-O-eicosanoylglucosid, Butyl-6-O-docosanoylglucosid, Butyl-6-O-cis-9-octadecanoylglucosid, Butyl6-O-cis,cis-9,12-octadecanoylglucosid oder Butyl-6-O-cis,cis,cis-9,12,15-octadecatrienoylglucosid sind.

23. Ein Produkt, das mehr als 80%, vorzugsweise mehr als 90%, am bevorzugtesten mehr als 95%, einer Verbindung von Formel I nach einem der Ansprüche 14 - 22 enthält.

24. Ein Reinigungsmittel, das eine Verbindung von Formel I nach einem der Ansprüche 14 - 22 enthält.

## Revendications

1. Procédé de préparation de composés de formule I
R-COO-X-OR¹ (I)
dans laquelle R¹ représente un alkyle de 2 à 6 atomes de carbone ou R¹ représente l'un des groupes 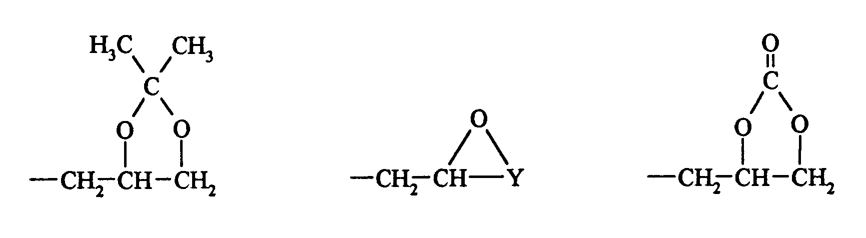 dans lesquels Y représente un méthylène ou un éthylène,
X représente un reste de monosaccharide portant le groupe OR¹ sur l'atome de carbone anomère et portant un groupe RCOO- sur un groupe hydroxy primaire, et
R représente un alkyle de 4 à 24 atomes de carbone,
par condensation d'un acide ou d'un ester de formule générale II
R-COOR² (II)
dans laquelle R est tel qu'indiqué ci-dessus, et R² représente un hydrogène ou un alkyle inférieur, avec un glycoside de formule générale III
HO-X-OR¹ (III)
dans laquelle X et R¹ sont chacun tels que définis ci-dessus, en présence d'un catalyseur, caractérisé en ce que ledit catalyseur est une hydrolase.

2. Procédé selon la revendication 1, dans lequel le monosaccharide correspondant au reste X porte le groupe -OR¹ sur l'atome de carbone anomère terminal.

3. Procédé selon la revendication 1 ou 2, dans lequel R¹ est un groupe alkyle non substitué.

4. Procédé selon l'une quelconque des revendications précédentes, dans lequel R¹ contient 2,3 ou 4 atomes de carbone.

5. Procédé selon l'une quelconque des revendications précédentes, dans lequel R¹ est un reste éthyle, propyle, isopropyle ou butyle, de préférence éthyle ou isopropyle.

6. Procédé selon l'une quelconque des revendications précédentes, dans lequel le monosaccharide correspondant au reste X est le glucose, le fructose, le ribose, le mannose ou le galactose, de préférence le glucose ou le galactose.

7. Procédé selon l'une quelconque des revendications précédentes, dans lequel l'acide gras correspondant au reste R-COO- contient 6 à 22 atomes de carbone.

8. Procédé selon l'une quelconque des revendications précédentes, dans lequel l'acide gras est l'acide hexanoïque, l'acide heptanoïque, l'acide octanoïque, l'acide nonanoïque, l'acide décanoïque, l'acide dodécanoïque, l'acide tétradécanoïque, l'acide hexadécanoïque, l'acide octadécanoïque, l'acide eicosanoïque, l'acide docosanoïque, l'acide cis-9-octadécanoïque, l'acide cis,cis-9,12-octadécanoïque ou l'acide cis,cis,cis-9,12,15-octadécatriénoïque.

9. Procédé selon la revendication 8, dans lequel R¹ est un reste éthyle, isopropyle, propyle ou butyle, et X est un reste de glucose.

10. Procédé selon l'une quelconque des revendications précédentes, caractérisé en ce que l'hydrolase est une lipase, une estérase ou une protéase.

11. Procédé selon l'une quelconque des revendications précédentes, caractérisé en ce que la lipase peut être produite par des espèces de *Mucor*, *Humicola*, *Pseudomonas* ou *Candida.*

12. Procédé selon l'une quelconque des revendications précédentes, caractérisé en ce que l'enzyme est utilisée sous forme immobilisée.

13. Procédé selon l'une quelconque des revendications précédentes, caractérisé en ce que l'on n'ajoute pas de solvant.

14. Composés de formule générale I
R-COO-X-OR¹ (I)
dans laquelle R¹ représente un alkyle de 2 à 6 atomes de carbone ou R¹ représente l'un des groupes 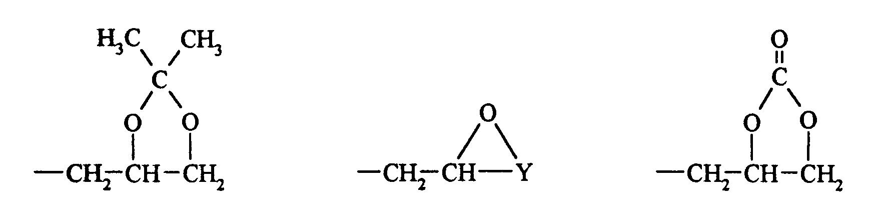 dans lesquels Y représente un méthylène ou un éthylène,
X représente un reste de monosaccharide portant le groupe OR¹ sur l'atome de carbone anomère et portant un groupe RCOO- sur un groupe hydroxy primaire, et
R représente un alkyle de 4 à 24 atomes de carbone.

15. Composés selon la revendication 14, dans lesquels le monosaccharide correspondant au reste X porte le groupe -OR¹ sur l'atome de carbone anomère terminal.

16. Composés selon la revendication 14 ou 15 dans lesquels R¹ est un groupe alkyle non substitué.

17. Composés selon l'une quelconque des revendications précédentes, dans lesquels R¹ contient 2,3 ou 4 atomes de carbone.

18. Composés selon l'une quelconque des revendications précédentes, dans lesquels R¹ est un reste éthyle, propyle, isopropyle ou butyle, de préférence éthyle ou isopropyle.

19. Composés selon l'une quelconque des revendications précédentes, dans lesquels le monosaccharide correspondant au reste X est le glucose, le fructose, le ribose, le mannose ou le galactose, de préférence le glucose ou le galactose.

20. Composés selon l'une quelconque des revendications 16 à 19, dans lesquels l'acide gras correspondant au reste R-COO- contient 6 à 22 atomes de carbone.

21. Composés selon la revendication 20, dans lesquels l'acide gras est l'acide hexanoïque, l'acide heptanoïque, l'acide octanoïque, l'acide nonanoïque, l'acide décanoïque, l'acide dodécanoïque, l'acide tétradécanoïque, l'acide hexadécanoïque, l'acide octadécanoïque, l'acide eicosanoïque, l'acide docosanoïque, l'acide cis-9-octadécanoïque, l'acide cis,cis-9,12-octadécanoïque ou l'acide cis,cis,cis-9,12,15-octadécatriénoïque.

22. Composés selon la revendication 14, qui sont le 6-O-hexanoylglucoside d'éthyle, le 6-O-heptanoylglucoside d'éthyle, le 6-O-octanoylglucoside d'éthyle, le 6-O-nonanoylglucoside d'éthyle, le 6-O-décanoylglucoside d'éthyle, le 6-O-dodécanoylglucoside d'éthyle, le 6-O-tétradécanoylglucoside d'éthyle, le 6-O-hexadécanoylglucoside d'éthyle, le 6-O-octadécanoylglucoside d'éthyle, le 6-O-eicosanoylglucoside d'éthyle, le 6-O-docosanoylglucoside d'éthyle, le 6-O-cis-9-octadécanoylglucoside d'éthyle, le 6-O-cis,cis-9,12-octadécanoylglucoside d'éthyle, le 6-O-cis,cis,cis-9,12,15-octadécatriénoylglucoside d'éthyle, le 6-O-hexanoylglucoside d'isopropyle, le 6-O-heptanoylglucoside d'isopropyle, le 6-O-octanoylglucoside d'isopropyle, le 6-O-nonanoylglucoside d'isopropyle, le 6-O-décanoylglucoside d'isopropyle, le 6-O-dodécanoylglucoside d'isopropyle, le 6-O-tétradécanoylglucoside d'isopropyle, le 6-O-hexadécanoylglucoside d'isopropyle, le 6-O-octadécanoylglucoside d'isopropyle, le 6-O-eicosanoylglucoside d'isopropyle, le 6-O-docosanoylglucoside d'isopropyle, le 6-O-cis-9-octadécanoylglucoside d'isopropyle, le 6-O-cis,cis-9,12-octadécanoylglucoside d'isopropyle, le 6-O-cis,cis,cis-9,12,15-octadécatriénoylglucoside d'isopropyle, le 6-O-hexanoylglucoside de propyle, le 6-O-heptanoylglucoside de propyle, le 6-O-octanoylglucoside de propyle, le 6-O-nonanoylglucoside de propyle, le 6-O-décanoylglucoside de propyle, le 6-O-dodécanoylglucoside de propyle, le 6-O-tétradécanoylglucoside de propyle, le 6-O-hexadécanoylglucoside de propyle, le 6-O-octadécanoylglucoside de propyle, le 6-O-eicosanoylglucoside de propyle, le 6-O-docosanoylglucoside de propyle, le 6-O-cis-9-octadécanoylglucoside de propyle, le 6-O-cis,cis-9,12-octadécanoylglucoside de propyle, le 6-O-cis,cis,cis-9,12,15-octadécatriénoylglucoside de propyle, le 6-O-hexanoylglucoside de butyle, le 6-O-heptanoylglucoside de butyle, le 6-O-octanoylglucoside de butyle, le 6-O-nonanoylglucoside de butyle, le 6-O-décanoylglucoside de butyle, le 6-O-dodécanoylglucoside de butyle, le 6-O-tétradécanoylglucoside de butyle, le 6-O-hexadécanoylglucoside de butyle, le 6-O-octadécanoylglucoside de butyle, le 6-O-eicosanoylglucoside de butyle, le 6-O-docosanoylglucoside de butyle, le 6-O-cis-9-octadécanoylglucoside de butyle, le 6-O-cis,cis-9,12-octadécanoylglucoside de butyle ou le 6-O-cis,cis,cis-9,12,15-octadécatriénoylglucoside de butyle.

23. Produit contenant plus de 80 %, de préférence plus de 90 %, de façon la plus préférée plus de 95 %, d'un composé de formule I selon l'une quelconque des revendications 14 à 22.

24. Agent de nettoyage contenant un composé de formule I selon l'une quelconque des revendications 14 à 22.
